# EUROPEAN PATENT APPLICATION

(11) **EP 4 310 068 A1**
(43) Date of publication of application: **24.01.2024**
(21) Application number: 22185843.4
(22) Date of filing: 19.07.2022
(51) Int. Cl.: C07C 41/01, C07C 43/04, C01B 3/00

(54) **ONE-STEP PROCESS FOR PRODUCING DIMETHYL ETHER**

(71) Applicant: Nederlandse Organisatie voor toegepast- natuurwetenschappelijk onderzoek TNO, 2595 DA 's-Gravenhage (NL)
(72) Inventor: The designation of the inventor has not yet been filed
(74) Representative: Nederlandsch Octrooibureau

(57) **Abstract**

The invention concerns a process for the conversion of ammonia (NH₃) and COₓ, wherein x = 1-2, to dimethyl ether (DME) wherein a feed comprising NH₃ and COₓ, wherein x = 1-2, is provided to a reactor to obtain a reaction product comprising DME, wherein the reactor comprises a catalyst for converting NH₃ to nitrogen (N₂) and hydrogen (H₂), a catalyst for converting H₂ and COₓ, wherein x = 1-2, to DME, and a sorbent material capable of adsorbing water. The invention furthermore concerns a composition a catalyst for converting NH₃ to nitrogen (N₂) and hydrogen (H₂), a catalyst for converting H₂ and COₓ, wherein x = 1-2, to DME, and a sorbent material capable of adsorbing water and the use of that composition in the conversion of ammonia (NH₃) and COₓ, wherein x = 1-2, to dimethyl ether (DME).

## Description

### Introduction

Dimethyl ether (DME) is a valuable compound useful in the chemical industry, e.g. as precursor of dimethyl sulphate, acetic acid or for olefin production. It is an important research chemical and is used as refrigerant and propellant. Moreover, DME may find more widespread use in the future, as it is being developed as novel fuel, e.g. as replacement for or additive to propane in LPG and as diesel fuel additive. DME can be produced by catalytic hydrogenation of CO, e.g. using synthesis gas (a fuel gas mixture consisting primarily of hydrogen and carbon monoxide). This can be done directly or by first converting syngas into methanol. The direct method of producing of DME from syngas involves contacting the synthesis gas with a bifunctional catalyst comprising a methanol synthesis catalyst and a methanol dehydration catalyst, such that isolation and purification of the methanol is not required.

A reaction scheme for the synthesis of dimethyl ether from syngas has been developed:

| | | | |
|---|---|---|---|
| Methanol synthesis: | CO₂ + 3 H₂ | ↔ | CH₃OH + H₂O |
| Water gas shift: | CO + H₂O | ↔ | H₂ + CO₂ |
| Methanol dehydration: | 2 CH₃OH | ↔ | CH₃OCH₃ + H₂O |
| Overall: | 3 CO + 3 H₂ | ↔ | CH₃OCH₃ (DME) + CO₂ |

As syngas typically comes from non-renewable sources, alternatives raw material are considered to produce DME. One of the options is to make use of CO₂, such as obtained from CO₂ capture (Mota et al., Catalysts 11, 411, 2021). CO₂ can be combined with H₂ and converted into DME, the overall reaction equation is:

2 CO₂ + 6 H₂ ↔ CH₃OCH₃ (DME) + 3 H₂O

For DME synthesis typically a combination of CO and CO₂ is used for feedstock. Both the conversion of CO and CO₂ together with H₂ are equilibrium reactions. The reaction efficiency can be improved by capturing the water by a sorbent capable of adsorbing water as is described in WO 2017/121817 and by Van Kampen et al. (Reaction Chemistry & Engineering, 6(2), 244-257 (2021)). The process of WO 2017/121817 and Van Kampen et al. is named the SEDMES, sorption enhanced dimethyl ether synthesis. The reaction preferably is carried out at elevated pressure up to 200 bara and relative low temperatures, around 250°C.

The H₂ for the SEDMES process can origin for example from electrolysis of water, using green electricity. A disadvantage of H₂ is that storage and transport is not straightforward as the volumetric energy density is low. Therefore other options to store and transport H₂ are considered. One of these options is ammonia NH₃ (Lucentini et al., Ind. Eng. Chem. Res., 60, 18560-18611, 2021). Ammonia can be cracked to deliver H₂ and N₂. The H₂ can subsequently be used in the SEDMES process. The use of NH₃ to deliver H₂ for the SEDMES process has not been described in the art. One of the reasons may be that cracking of NH₃ is inefficient as after cracking a gaseous mixture of H₂ and N₂ needs to be separated. Without separation the reactant density during SME synthesis is low as in the above reaction equation next to 6 molecules H₂ also 2 molecules of N₂ are present. Moreover a larger volume of gas needs to be pressurized, which is energy inefficient. NH₃ cracking is typically carried out at temperatures above 500 °C, preferably around 800 - 900 °C and at ambient pressure.

Therefore there is need for a process for producing DME that makes use of renewable sources and that provides for an efficient conversion of the renewable sources.

### Summary of the invention

The inventors now found that NH₃ cracking and DME synthesis can be carried out in a one-step process. In this one step process a catalyst for converting NH₃ to nitrogen (N₂) and hydrogen (H₂), a catalyst for converting H₂ and CO_{X}, wherein x = 1-2, to DME and a sorbent material capable of adsorbing water is present. This process has several benefits.

First of all, due to the integration of NH₃ cracking and DME synthesis only one reactor is needed instead of a separate cracking reactor and a separate DME synthesis reactor. The capital expenditure and operations costs therefore will be lower. In addition due to the process integration the heat generated during DME synthesis can be in situ transferred to the endothermic cracking reaction, instead that the heat needs to be transferred through a less efficient heat exchanger circuit. No heat will be lost. Also the risk for a run-away of the highly exothermic DME synthesis reaction needs less effort to mediate as the in-situ heat transfer tempers the heat development in the reactor.

As is demonstrated by the inventors conventional NH₃ cracking requires a very high temperature and a low pressure to reach a sufficiently high conversion. The endothermic nature of the cracking reaction in combination with a positive entropy is debit for this. Surprisingly in the process of the invention the temperature can be lowered considerably whilst still reaching full conversion for the NH₃ cracking. The inventors believe that by in situ coupling of H₂ generation (NH₃ cracking) with H₂ consumption (DME synthesis) the cracking reaction is pulled towards full conversion, even at lower temperatures. The lower temperature of the cracking reaction provides for less energy loss.

For conventional DME synthesis with CO and/or CO₂ as feedstock, the H₂ needs to be provided in pressurized form as the DME synthesis takes place at elevated pressure. Pressurization of gasses such as H₂ is far more expensive and energy inefficient than pressurization of liquids. Liquid pressurization 'only' needs a pump whilst gas pressurization requires elaborate compressors. A surprising benefit of the invention is also that no longer H₂ gas needs to be pressurized, but only liquid NH₃. For conventional NH₃ cracking such pressurized NH₃ is undesirable, as it implies a higher pressure for the cracking reaction which affects conversion negatively. However, same as for the temperature, by in situ coupling of H₂ generation (NH₃ cracking) with H₂ consumption (DME synthesis) the cracking reaction is pulled towards full conversion, even at increased pressure.

A third surprising advantage of the in situ coupling of H₂ generation (NH₃ cracking) with H₂ consumption (DME synthesis) is that a recycle stream that is present in conventional NH₃ cracking is no longer needed. In conventional cracking the reaction product is deprived of remnant NH₃. Remnant NH₃ is inevitably present due to economic considerations with regards to the equilibrium nature of the reaction. It is economically unattractive to aim for very high conversion. This remnant NH₃ is separated for the N₂ and H₂ mixture and fed back to the reactor, which evidently requires CAPEX, OPEX and energy. In addition cumbersome gas-gas separation of N₂ and H₂ can be replaced by straightforward liquid-gas separation of DME and N₂. The gas-gas separation requires even more CAPEX, OPEX and energy compared to the NH₃ recycle.

All in all by the current invention of integration NH₃ cracking with DME synthesis several issues that confine a widespread use of NH₃ as H₂ carrier are resolved. Conventionally such integration would not be considered due to the very different reaction temperatures and pressures of the two reactions. Anyhow the prior art does not suggest to combine NH₃ cracking with DME synthesis from CO and/or CO₂, even not in a two-step process. Moreover the prediction of the outcome of a reaction with many reactive species and an adsorption step included is not straightforward, a priori a skilled person could not predict the outcome. The inventors now demonstrated in the example that the reaction surprisingly leads to a high DME conversion, leading to the benefits as explained.

### Detailed Description

In a first embodiment the invention concerns a process for the conversion of ammonia (NH₃) and CO_{X}, wherein x = 1-2, to dimethyl ether (DME) wherein in a reaction step a) a feed comprising NH₃ and CO_{X}, wherein x = 1-2, is provided to a reactor to obtain a reaction product comprising DME, wherein the reactor comprises a catalyst for converting NH₃ to nitrogen (N₂) and hydrogen (H₂), a catalyst for converting H₂ and CO_{X}, wherein x = 1-2, to DME, and a sorbent material capable of adsorbing water.

The invention furthermore concerns a composition comprising a catalyst for converting NH₃ to N₂ and H₂, a catalyst for converting H₂ and CO_{X}, wherein x = 1-2, to DM E and a sorbent material capable of adsorbing water.

Furthermore the invention concerns the use of a composition comprising a catalyst for converting NH₃ to N₂ and H₂, a catalyst for converting H₂ and CO_{X}, wherein x = 1-2, to DME and a sorbent material capable of adsorbing water for converting ammonia (NH₃) and CO_{X}, wherein x = 1-2, to dimethylether (DME), preferably wherein the use involves one or more of the following:
- the conversion is increased compared to when the sorbent material is not present,
- the reaction rate is increased compared to when the sorbent material is not present, or
- the carbon selectivity towards DME is increased compared to when the sorbent material is not present.

The invention furthermore concerns a reactor comprising a catalyst for converting NH₃ to N₂ and H₂, a catalyst for converting H₂ and CO_{X}, wherein x = 1-2, to DME and a sorbent material capable of adsorbing water, wherein preferably the reactor is a fixed bed reactor or a fluidized bed reactor, more preferably the reactor is a fluidized bed reactor.

The embodiments disclosed in this document apply to all aspects of the invention, i.e. the process for the conversion of ammonia (NH₃) and CO_{X}, wherein x = 1-2, to dimethyl ether (DME); the composition comprising a catalyst for converting NH₃ to N₂ and H₂, a catalyst for converting H₂ and CO_{X}, wherein x = 1-2, to DME and a sorbent material capable of adsorbing water; the use of a composition comprising a catalyst for converting NH₃ to N₂ and H₂, a catalyst for converting H₂ and CO_{X}, wherein x = 1-2, to DME and a sorbent material capable of adsorbing water for converting ammonia (NH₃) and CO_{X}, wherein x = 1-2, to dimethylether (DME) and the reactor comprising a catalyst for converting NH₃ to N₂ and H₂, a catalyst for converting H₂ and CO_{X}, wherein x = 1-2, to DME and a sorbent material capable of adsorbing water.

### NH₃ cracking

NH₃ cracking is known in the art and concerns converting NH₃ in N₂ and H₂. NH₃ is named ammonia. NH₃ cracking is named alternatively decomposition of ammonia. NH₃ cracking is typically carried out in the presence of a catalyst for converting NH₃ to N₂ and H₂. This is generally done at low (atmospheric) pressure and high temperature (400 °C and higher, preferably at 800°C - 900°C), for enthalpic and entropic reasons. Although a high temperature is preferred, in the art catalysts for converting NH₃ to N₂ and H₂ are known that work at lower temperatures. Ru/support catalysts are still functional at 350 (327) °C; Ru-alloys catalysts are still functional at 300 °C (Lucentini et al., Industrial & Engineering Chemistry Research, 60 (51), 18560-18611 (2021)). Other authors such as Itoh et al. (Materials Transactions, 43 (11), 2763-2767 (2002)) and Hill et al. (Applied Catalysis B: Environmental, 172, 129-135 (2015)) demonstrate catalyst activity at temperatures down to 200 °C.

In a preferred embodiment the catalyst for converting NH₃ to N₂ and H₂ is a catalyst that demonstrate catalytic activity for converting NH₃ to N₂ and H₂ at temperatures in the range of 200 °C - 300 °C. In a preferred embodiment the catalyst for converting NH₃ to N₂ and H₂ is a metal based catalyst, preferably the metal is selected from the list consisting of ruthenium, nickel, platinum, tantalum, rhodium, tungsten, iridium, cobalt, cupper, iron, chromium, lead, lithium, manganese, vanadium, palladium, molybdenum, cerium, aluminum, strontium, zirconium, sodium, potassium, cesium and combinations thereof, more preferably the metal is ruthenium, nickel or iron, optionally combined with smaller amounts of other metals, most preferably the metal is ruthenium optionally combined with smaller amounts of other metals. Preferably the metal based catalyst is a metal/support catalyst, a metal alloy catalyst, a metal composite catalyst and/or a metal oxide catalyst wherein preferably the metal is selected from the list consisting of ruthenium, nickel, platinum, tantalum, rhodium, tungsten, iridium, cobalt, cupper, iron, chromium, lead, lithium, manganese, vanadium, palladium, molybdenum, cerium, aluminum, strontium, zirconium, sodium, potassium, cesium and combinations thereof, more preferably the metal is ruthenium, nickel or iron, most preferably the metal is ruthenium. Preferably the support comprises carbon nanotubes. In a preferred embodiment the catalyst for converting NH₃ to N₂ and H₂ is a ruthenium/support catalyst, preferably wherein the support comprises carbon nanotubes, or an iron-rare earth oxide composite catalyst, preferably wherein the rare earth is cerium.

In a preferred embodiment the catalyst for converting NH₃ to N₂ and H₂ has a maximum water adsorption capacity of less than 0.10 gram water per gram catalyst at the temperature and pressure in step a), more preferably less than 0.04 gram water per gram catalyst at the temperature and pressure in step a), even more preferably less than 0.02 gram water per gram catalyst at the temperature and pressure in step a), most preferably less than 0.01 gram water per gram catalyst at the temperature and pressure in step a).

The water adsorption capacity preferably is measured through breakthrough analysis or through thermogravimetric analysis (TGA), preferably through TGA. Both methods are explained in Van Kampen et al. (Adsorption 27, 577-589 (2021)) and Ruthven (Ruthven, D. M. (1984). Principles of adsorption and adsorption processes. John Wiley & Sons.).

In breakthrough analysis, a sample of the catalyst first is dried at 400°C in an atmosphere not comprising water vapour, subsequently at the temperature and pressure in step a) the catalyst is exposed to a gas mixture comprising 40 mol% water and an inert tracer until equilibrated, wherein time integration of the measured outlet composition yields the amount of water adsorbed. The amount of water adsorbed expressed in weight can be divided by the weight of the catalyst sample to deliver the water adsorption capacity in gram water per gram catalyst.

In thermogravimetric analysis a sample of the catalyst first is dried at a temperature of 400°C in an atmosphere not comprising water vapour, subsequently the catalyst is exposed to a gas mixture comprising 40 mol% water for at least 1000 seconds at the temperature and pressure in step a), and the weight increase of the dried catalyst between before and after exposure to the gas mixture comprising 40 mol% water is measured. The water adsorption capacity is the weight increase divided by the weight of the dried catalyst sample.

The form of the catalyst for converting NH₃ to N₂ and H₂ depends on the reactor size and type. In a preferred embodiment the catalyst is a particulate material, preferably the particle size is between 0.10 millimetre and 10 centimetre, more preferably between 0.30 millimetre and 5.0 centimetre, even more preferably between 1.0 centimetre and 5.0 centimetre. Typically the optimal particle size for a fluidized bed reactor is smaller compared to a fixed bed reactor; typically the optimal particle size for an industrial scale reactor is larger than that of a lab reactor. The skilled person is able to select the optimal particle size for a specific reactor design.

### DME synthesis from H₂ and CO_{X}, wherein x = 1-2

CO_{X} denotes CO, CO₂ or mixtures thereof, and may also be referred to as "carbon oxide", with CO and CO₂ being the two carbon oxide species. CO is carbon monoxide and CO₂ is carbon dioxide. The value of x denotes the number of oxygen atoms present per carbon atom in the carbon oxide fraction (i.e. CO + CO₂) of the feedstock, irrespective of any further oxygen and/or carbon atoms that may be present in the feedstock. Thus, x is in the range of 1 - 2, wherein x = 1 indicates pure CO and x = 2 indicates pure CO₂. An intermediate value for x indicates that a mixture of CO and CO₂ is present in the feedstock, which can readily be determined by the skilled person. As example, when x = 1.9, 1.9 oxygen atoms are present per carbon atom, meaning that the molar ratio of CO:CO₂ is 1:9. Likewise, when x = 1.5, the CO:CO₂ molar ratio is 1:1. In a preferred embodiment x = 2.

The optimal molar ratio of NH₃ to CO_{X} in the feedstock depends mainly on the value of x. Theoretically, based on the stoichiometry of the reaction, the optimal NH₃ to CO_{X} molar ratio ranges from 4 : 3 when x = 1 to 2 : 1 when x = 2. Although stoichiometric amounts of NH₃ and COx are preferred, the process according to the invention also runs smoothly with super-stoichiometric and even slight sub-stoichiometric amounts of NH₃.

Dimethyl ether (DME) is also named methoxymethane. In the DME synthesis from H₂ and CO_{X}, wherein x = 1-2, both the methanol synthesis reaction as the methanol dehydration reaction occurs. DME synthesis from H₂ and CO_{X}, wherein x = 1-2, is carried out in the presence of a catalyst for converting H₂ and CO_{X}, wherein x = 1-2, to DME. Catalysts for this reaction typically comprise two functionalities; for the methanol synthesis metal sites are required whilst for the methanol dehydration acid sites are required, therefore the catalyst is bifunctional. Catalyst for converting H₂ and CO_{X}, wherein x = 1-2, to DME are known in the art (Mota et al., Catalysts 11, 411, 2021). Van Kampen et al. (Reaction Chemistry & Engineering, 6(2), 244-257 (2021)) disclose catalysts that demonstrate proficient catalytic activity for DME synthesis in the range of 225-300 °C.

In a preferred embodiment the catalyst for converting H₂ and CO_{X}, wherein x = 1-2, to DME is a catalyst that demonstrate catalytic activity for converting H₂ and CO_{X}, wherein x = 1-2, to DME at temperatures in the range of 200 °C - 300 °C. In a preferred embodiment the catalyst for converting H₂ and CO_{X}, wherein x = 1-2, to DME is selected from a bifunctional catalyst for converting H₂ and CO_{X}, wherein x = 1-2, to DME or a combination of a methanol synthesis catalyst and a methanol dehydration catalyst. A methanol synthesis catalyst is a catalyst that catalyses the synthesis of methanol from CO_{X}, wherein x =1-2, and H₂, a methanol dehydration catalyst is a catalyst that catalyses the dehydration of methanol into DME and H₂O. Any DME catalyst system known in the art is suitable to be used in this respect. Suitable catalysts include alumina containing copper/zinc oxide based catalysts (Cu-ZnO-Al₂O₃), but other catalysts are also suitable. In a preferred embodiment the catalyst for converting H₂ and CO_{X}, wherein x = 1-2, to DME is an alumina containing copper/zinc oxide based catalyst.

In a preferred embodiment the catalyst for converting H₂ and CO_{X}, wherein x = 1-2, to DME has a maximum water adsorption capacity of less than 0.10 gram water per gram catalyst at the temperature and pressure in step a), more preferably less than 0.04 gram water per gram catalyst at the temperature and pressure in step a), even more preferably less than 0.02 gram water per gram catalyst at the temperature and pressure in step a), most preferably less than 0.01 gram water per gram catalyst at the temperature and pressure in step a).

The water adsorption capacity preferably is measured through breakthrough analysis or through thermogravimetric analysis (TGA), preferably through TGA. Both methods are explained in Van Kampen et al. (Adsorption 27, 577-589 (2021)) and Ruthven (Ruthven, D. M. (1984). Principles of adsorption and adsorption processes. John Wiley & Sons.).

In breakthrough analysis, a sample of the catalyst first is dried at 400°C in an atmosphere not comprising water vapour, subsequently at the temperature and pressure in step a) the catalyst is exposed to a gas mixture comprising 40 mol% water and an inert tracer until equilibrated, wherein time integration of the measured outlet composition yields the amount of water adsorbed. The amount of water adsorbed expressed in weight can be divided by the weight of the catalyst sample to deliver the water adsorption capacity in gram water per gram catalyst.

In thermogravimetric analysis a sample of the catalyst first is dried at a temperature of 400°C in an atmosphere not comprising water vapour, subsequently the catalyst is exposed to a gas mixture comprising 40 mol% water for at least 1000 seconds at the temperature and pressure in step a), and the weight increase of the dried catalyst between before and after exposure to the gas mixture comprising 40 mol% water is measured. The water adsorption capacity is the weight increase divided by the weight of the dried catalyst sample.

The form of the catalyst for converting H₂ and CO_{X}, wherein x = 1-2, to DME depends on the reactor size and type. In a preferred embodiment the catalyst is a particulate material, preferably the particle size is between 0.10 millimetre and 10 centimetre, more preferably between 0.30 millimetre and 5.0 centimetre, even more preferably between 1.0 centimetre and 5.0 centimetre. Typically the optimal particle size for a fluidized bed reactor is smaller compared to a fixed bed reactor; typically the optimal particle size for an industrial scale reactor is larger than that of a lab reactor. The skilled person is able to select the optimal particle size for a specific reactor design.

### The sorbent material capable of adsorbing water

Sorbent materials capable of adsorbing water are known in the art. Preferably the sorbent material capable of adsorbing water is a molecular sieve, more preferably a zeolite, even more preferably an LTA zeolite. A molecular sieve is a material comprising pores of uniform size. Zeolites are aluminosilicate minerals comprising pores of uniform size. An LTA zeolite is a Linde Type A zeolite. Preferably the sorbent material capable of adsorbing water is a molecular sieve having a pore diameter in the range of 2 - 50 ångström (Å), more preferably in the range of 2-10 Å, even more preferably in the range of 2-5 Å most preferably in the range of 3-4 Å. More preferably the sorbent material capable of adsorbing water is a zeolite having a pore diameter in the range of 2 - 50 Å, more preferably in the range of 2-10 Å, even more preferably in the range of 2-5 Å most preferably in the range of 3-4 Å. Even more preferably the sorbent material capable of adsorbing water is an LTA zeolite having a pore diameter in the range of 1 - 50 Å, more preferably in the range of 2-10 Å, even more preferably in the range of 2-5 Å most preferably in the range of 3-4 Å.

In a preferred embodiment the sorbent material capable of adsorbing water has a molar ratio of Si to Al ratio between 0.3 : 1.0 and 10.0 : 3.0, more preferably between 0.5:1.0 and 2.0:1.0, most preferably between 0.8:1.0 and 1.2:1.0. An equal amount of Si to Al points to a less acid structure that is well capable of adsorbing water. Preferably the sorbent material capable of adsorbing water has a water adsorption capacity of at least 0.04 gram water per gram sorbent material at the temperature and pressure in step a), more preferably at least 0.06 gram water per gram sorbent material at the temperature and pressure in step a), even more preferably at least 0.08 gram water per gram sorbent material at the temperature and pressure in step a), most preferably at least 0.10 gram water per gram sorbent material at the temperature and pressure in step a).

The water adsorption capacity preferably is measured through breakthrough analysis or through thermogravimetric analysis (TGA), preferably through TGA. Both methods are explained in Van Kampen et al. (Adsorption 27, 577-589 (2021)) and Ruthven (Ruthven, D. M. (1984). Principles of adsorption and adsorption processes. John Wiley & Sons.).

In breakthrough analysis, a sample of the sorbent material capable of adsorbing water first is dried at 400°C in an atmosphere not comprising water vapour, subsequently at the temperature and pressure in step a) the sorbent material capable of adsorbing water is exposed to a gas mixture comprising 40 mol% water and an inert tracer until equilibrated, wherein time integration of the measured outlet composition yields the amount of water adsorbed. The amount of water adsorbed expressed in weight can be divided by the weight of the sorbent material capable of adsorbing water sample to deliver the water adsorption capacity in gram water per gram sorbent material capable of adsorbing water.

In thermogravimetric analysis a sample of the sorbent material capable of adsorbing water first is dried at a temperature of 400°C in an atmosphere not comprising water vapour, subsequently the sorbent material capable of adsorbing water is exposed to a gas mixture comprising 40 mol% water for at least 1000 seconds at the temperature and pressure in step a), and the weight increase of the dried sorbent material capable of adsorbing water between before and after exposure to the gas mixture comprising 40 mol% water is measured. The water adsorption capacity is the weight increase divided by the original weight of the dried sorbent material capable of adsorbing water sample.

The form of the sorbent material capable of adsorbing water depends on the reactor size and type. In a preferred embodiment the sorbent material capable of adsorbing water is a particulate material, preferably the particle size is between 0.10 millimetre and 10 centimetre, more preferably between 0.30 millimetre and 5.0 centimetre, even more preferably between 1.0 centimetre and 5.0 centimetre. Typically the optimal particle size for a fluidized bed reactor is smaller compared to a fixed bed reactor; typically the optimal particle size for an industrial scale reactor is larger than that of a lab reactor. The skilled person is able to select the optimal particle size for a specific reactor design.

In a preferred embodiment at the start of reaction step a) the sorbent material capable of adsorbing water comprises less than 0.10 gram water per gram sorbent material at the temperature and pressure in step a), even more preferably less than 0.04 gram water per gram sorbent material at the temperature and pressure in step a), even more preferably less than 0.02 gram water per gram sorbent material at the temperature and pressure in step a), most preferably less than 0.01 gram water per gram sorbent material at the temperature and pressure in step a).

### Combination of a catalyst for converting NH₃ to N₂ and H₂, a catalyst for converting H₂ and CO_{X}, wherein x = 1-2, to DME, and a sorbent material capable of adsorbing water

The invention furthermore concerns a combination of a catalyst for converting NH₃ to N₂ and H₂, a catalyst for converting H₂ and CO_{X}, wherein x = 1-2, to DME, and a sorbent material capable of adsorbing water. The catalyst for converting NH₃ to N₂ and H₂, the catalyst for converting H₂ and CO_{X}, wherein x = 1-2, to DME, and a sorbent material capable of adsorbing water are not the same. Preferably this combination is comprised in a reactor, preferably a fixed bed reactor or a fluidized bed reactor. In a preferred embodiment the catalyst for converting NH₃ to N₂ and H₂, the catalyst for converting H₂ and CO_{X}, wherein x = 1-2, to DME and the sorbent material capable of adsorbing water are not bound to each other. Binding may decrease the catalytic activity of the catalysts and/or the water adsorption capacity of the sorbent material. Moreover binding is expensive and provides for less freedom in selecting the ratio of catalysts and sorbent material capable of adsorbing water.

In a preferred embodiment the combination comprises in the range of 20 - 90 wt.% of the sorbent material capable of adsorbing water based on total of the catalyst for converting NH₃ to N₂ and H₂, the catalyst for converting H₂ and CO_{X}, wherein x = 1-2, to DME, and the sorbent material capable of adsorbing water, more preferably in the range of 30 - 80 wt.%, even more preferably in the range of 40 - 70 wt.%. Preferably the combination comprises in the range of 10 - 50 wt.% of the catalyst for converting NH₃ to N₂ and H₂ and in the range of 10 - 50 wt.% of the catalyst for converting H₂ and CO_{X}, wherein x = 1-2, to DME on total of the catalyst for converting NH₃ to N₂ and H₂, the catalyst for converting H₂ and CO_{X}, wherein x = 1-2, to DME, and the sorbent material capable of adsorbing water, more preferably the combination comprises in the range of 20 - 40 wt.% of the catalyst for converting NH₃ to N₂ and H₂ and in the range of 20-40 wt.% of the catalyst for converting H₂ and CO_{X}, wherein x = 1-2, to DME on total of the catalyst for converting NH₃ to N₂ and H₂, the catalyst for converting H₂ and CO_{X}, wherein x = 1-2, to DME, and the sorbent material capable of adsorbing water. In a preferred embodiment the combination comprises in the range of 20 - 90 wt.% of the sorbent material capable of adsorbing water and in the range of 10-50 wt.% of the catalyst for converting NH₃ to N₂ and H₂ and in the range of 10 - 50 wt.% of the catalyst for converting H₂ and CO_{X}, wherein x = 1-2, to DME based on total of the catalyst for converting NH₃ to N₂ and H₂, the catalyst for converting H₂ and CO_{X}, wherein x = 1-2, to DME, and the sorbent material capable of adsorbing water, more preferably the combination comprises in the range of 40 - 70 wt.% of the sorbent material capable of adsorbing water and in the range of 20 - 40 wt.% of the catalyst for converting NH₃ to N₂ and H₂ and in the range of 20 - 40 wt.% of the catalyst for converting H₂ and CO_{X}, wherein x = 1-2, to DME based on total of the catalyst for converting NH₃ to N₂ and H₂, the catalyst for converting H₂ and CO_{X}, wherein x = 1-2, to DME, and the sorbent material capable of adsorbing water.

### The process for the conversion of ammonia (NH₃) and CO_{X}, wherein x = 1-2, to dimethyl ether (DME)

The process for the conversion of ammonia (NH₃) and CO_{X}, wherein x = 1-2, to dimethyl ether (DME) comprises a reaction step a). In a preferred embodiment the reaction temperature is in the range of 150°C-400°C, more preferably in the range of 200°C - 300°C. A too low reaction temperature results in a low reaction rate, whilst a too high reaction temperature may affect water adsorption by the sorbent capable of adsorbing water in a negative way.

In a preferred embodiment the reaction pressure is in the range of 1 - 100 bara, preferably in the range of 20 - 80 bara, even more preferably in the range of 30 - 70 bara. A high pressure is advantageous for entropic reasons. The entropy of the reaction product is lower than that of the starting materials, and therefore a high pressure is advantageous for reaching a high conversion (Le Chatelier's principle). In addition, in case the reaction step a) is followed by a sorbent regeneration step b) a higher pressure provides for that the pressure can be reduced considerably in step b) which is advantageous for sorbent regeneration. A higher pressure requires more CAPEX and OPEX costs, therefore a too high pressure is not cost efficient.

The process for the conversion of ammonia (NH₃) and CO_{X}, wherein x = 1-2, to dimethyl ether (DME) is carried out in a reactor. In a preferred embodiment the reactor is a fixed bed reactor or a fluidized bed reactor, more preferably the reactor is a fluidized bed reactor.

Reactors comprising solid catalysts and solid sorbent material and having a gaseous feed and typically are not ideally mixed, their mixing behaviour is closer to plug flow. Due to the plug flow character the gas composition in the reactor differs between the reaction zone after the inlet of the reactor and the reaction zone before the outlet of the reactor. At the inlet of the reactor there may be a suboptimal ratio of reactants; in case the feed for step a) comprises NH₃ and COx, wherein x = 1-2, the reverse water-gas shift reaction and methanol synthesis reaction may not or limitedly occur in the reaction zone directly after the inlet due to that H₂ is not yet formed. In a preferred embodiment the feed in reaction step a) comprises H₂, preferably the feed comprises in the range of 1-20 mol% of H₂ on total of the feed, more preferably in the range of 4- 10 mol% of H₂ on total of the feed. In this way the reverse water-gas shift reaction and methanol synthesis reaction also occurs in the reaction zone directly after the inlet of the reactor which provides for a more efficient use of the reactor volume. In a preferred embodiment the H₂ is generated by partial NH₃ cracking in a separate reactor, preferably wherein the reaction product of the partial NH₃ cracking is comprised in the feed for reaction step a).

In a preferred embodiment for the process for the conversion of ammonia (NH₃) and COx, wherein x = 1-2, to dimethyl ether (DME) the carbon selectivity towards DME is at least 50%, more preferably at least 70%, even more preferably at least 90%, most preferably at least 95%. Carbon selectivity is known in the art, as for example described by Van Kampen et al., Reaction Chemistry & Engineering, 6(2), 244-257 (2021). Carbon selectivity is calculated on molar basis and concern the ratio of a specific carbon containing species over all the carbon containing species in a mixture. For the process for the conversion of ammonia (NH₃) and CO_{X}, wherein x = 1-2, to dimethyl ether (DME) the carbon selectivity equals 2·y(DME) / [ y(CO) + y(CO₂) + 2·y(DME) + y(methanol) + y(CH₄) ], wherein y(species) is the concentration of that species expressed in mol/m³ and wherein the carbon selectivity is expressed in percentage. The carbon selectivity of DME concerns the carbon selectivity of the reaction product of the process for the conversion of ammonia (NH₃) and COx, wherein x = 1-2, to dimethyl ether (DME).

Typically for adsorption enhanced reactions the sorbent material capable of adsorbing water needs to be regenerated. Regeneration of the sorbent material capable of adsorbing water concerns desorbing of water from the sorbent material. In this way the sorbent material is ready for adsorbing water in a next reaction step a). During the reaction step a) next to the formation of the reaction product also water is adsorbed by the sorbent material capable of adsorbing water.

In a preferred embodiment the process comprises a second step b) after step a) which is a sorbent regeneration step wherein the sorbent material is contacted with a purge gas. During sorbent regeneration water is desorbed from the sorbent material capable of adsorbing water. In a preferred embodiment the purge gas is air, N₂, H₂ or CO₂, more preferably air. Preferably the purge gas comprises less than 5.0 mol% water on total of the purge gas, preferably less than 1.0 mol% water, most preferably less than 0.5 mol% water. Preferably contacting with a purge gas is realized by feeding the purge gas to the reactor.

Preferably the process is operated cyclically wherein step b) is followed by step a). In this way a reactor of the process alternates between mode a) and mode b), optionally in combination with additional modes. Mode a) is the same as step a), mode b) is the same as step b). Considering for a manufacturing plant a continuous process is preferred the process preferably comprise two or more reactors, more preferably three or more reactors comprising the catalyst for converting NH₃ to N₂ and H₂, the catalyst for converting H₂ and CO_{X}, wherein x = 1-2, to DME and the sorbent material capable of adsorbing water. For this embodiment preferably at least one reactor of the two or three or more reactors is in mode a). By having two or more reactors, one reactor can be in reaction mode a) whilst the other reactor is in sorbent regeneration mode b). The moment the sorbent material capable of adsorbing water in the reactor that is in reaction mode a) is saturated with water the feed flow can be switched to the reactor that was in sorbent regeneration mode b) and the reaction can continue in this reactor; so this reactor goes into reaction mode a). Meanwhile the reactor comprising the water saturated sorbent material can go into sorbent regeneration mode b). In this way a continuous feed flow and product flow can be maintained. In some situations mode a) and mode b) are not of equal length. Therefore in a preferred embodiment the process comprises a step c) idle mode. During idle mode no gas is fed to the reactor.

Regeneration of a sorbent material capable of adsorbing water typically is carried out by contacting the sorbent material with a purge gas. To accelerate the desorption of water the temperature may be increased or the pressure may be lowered during contacting with the purge gas. Both temperature increase and pressure decrease stimulate the desorption of water. In a preferred embodiment the temperature in step b) is at least 100°C higher than the temperature of step a) and/or the pressure of step b) is at least 10 bar lower than the pressure of step a), more preferably the temperature in step b) is at least 150°C higher than the temperature of step a) and/or the pressure of step b) is at least 20 bar lower than the pressure of step a). In a preferred embodiment the pressure of step b) is at least 10 bar lower than the pressure of step a), more preferably the pressure of step b) is at least 20 bar lower than the pressure of step a).

### General Definitions

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". The word "about" or "approximately" when used in association with a numerical value (e.g. about 10) preferably means that the value may be the given value more or less 1% of the value.

The present invention has been described above with reference to a number of exemplary embodiments. Modifications and alternative implementations of some parts or elements are possible, and are included in the scope of protection as defined in the appended claims. All citations of literature and patent documents are hereby incorporated by reference.

Bara or bar(a) stands for bar absolute and concerns the pressure that is zero-referenced against a perfect vacuum. 1 bara is 100,000 N/m². 1 bara is 100,000 Pascal.

### Example 1 - Ammonia-fed adsorption-enhanced CO₂ conversion process

### Introduction

The example describes an ammonia-fed sorption-enhanced DME synthesis process. The process combines ammonia cracking:

| | | |
|---|---|---|
| 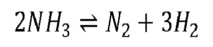 | ammonia cracking | (1) |

with CO₂ conversion, during which the following main reactions occur:

| | | |
|---|---|---|
| 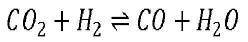 | reverse water-gas shift | (2) |
| 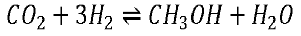 | methanol synthesis | (3) |
| 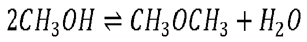 | methanol dehydration | (4) |
| 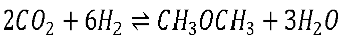 | DME synthesis | (5) |

Theses reaction equations represent the main reactions for the ammonia-fed sorption-enhanced DME synthesis. All reactions a equilibrium reactions, which can be performed with a finite conversion. By the principle of Le Chatelier, up to full conversions can be achieved by in situ separation of one of the reaction products (as for example is presented in WO2017121817 A1). The current example discussed the combination of (1) and (5) in the presence of a water adsorbent, to achieve the net reaction,

| | | |
|---|---|---|
| 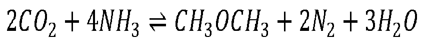 | direct DME synthesis from CO₂ | (6) |

Similarly, the same process can be performed with a carbon monoxide feed for which the same main reactions occur (1-5) as for CO₂ conversion:

| | | |
|---|---|---|
| 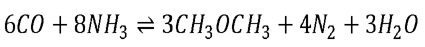 | direct DME synthesis from CO | (7) |

Actually, any mixture of CO₂ and CO can be used as feedstock, leading to a combination of (6) and (7). During the process, Reaction (2) is able to establish a local equilibrium between CO₂ and CO in the reactor at all times.

### Method

An equilibrium model was built in Aspen Plus, using the Peng-Robinson equation of state. The equilibrium constants are calculated by Aspen using Gibbs free energy minimisation. Rather than building a separation-enhanced reactor, the system was modelled using constructs: conventional unit operations that simulate the behaviour of the sorption-enhanced reaction process which is described by Schad (Chemical engineering progress, 94(1), 21-27, (1998)). Subsequent simulation of (i) a stoichiometric reactor, performing complete conversion according to reaction (6), i.e. producing the maximum amount of DME and H₂O, (ii) a separator, taking out a variable amount of H₂O from the reaction mixture, and (iii) an equilibrium reactor establishing the thermodynamic equilibrium according to a minimisation of the Gibbs free energy, i.e. producing back the reactants and side products according to all relevant equilibria; reactions 1-6. In this manner, the performance of a sorption-enhanced reaction can be simulated, allowing to calculate the resulting equilibrium composition as a function of the residual steam content.

### Results

The equilibrium composition as a function of the residual steam partial pressure is summarised in table 1. The residual steam is the amount of steam present after regeneration of the sorbent material. Without water removal, only some NH₃ is cracked to N₂ and H₂ while most CO₂ remains inert as witnessed by the minor amounts of CO, methanol (MeOH), and DME formed. Removing the water from the reaction mixture as a profound influence on the product composition. When no water is removed the starting materials CO₂ and NH₃ are present in high concentrations and only a minor amount of DME is formed (case A). Oppositely when 99.9% of the water is removed (case D) DME conversion is high and the reaction product hardly comprises CO₂ and NH₃.

**Table 1. Product composition for a stoichiometric feed of CO₂ and NH₃ at 250 °C and 50 bar(a), for three different degrees of water removal (all values in mol% on total of the composition).**

| **Case** | **A** | **B** | **C** | **D** |
|---|---|---|---|---|
| **Water removal** | 0.0% | 90.0% | 99.0% | 99.9% |
| **CO₂** | 24.4% | 2.85% | 0.001% | <0.001% |
| **CO** | 1.5% | 7.7% | 1.9% | 0.2% |
| **H₂** | 22.1% | 6.3% | 1.6% | 0.4% |
| **H₂O** | 4.5% | 0.029% | 0.00001% | <0.00001% |
| **NH₃** | 36.1% | 11.9% | 1.7% | 0.2% |
| **N₂** | 10.1% | 49.0% | 63.6% | 66.2% |
| **MeOH** | 0.504% | 0.208% | 0.003% | <0.001% |
| **DME** | 0.8% | 22.1% | 31.2% | 33.0% |

### Discussion and conclusion

As shown above, a reduction of the residual steam partial pressure by means of in situ adsorption produces a very favourable reaction equilibrium, that allows to produce in a single unit operation a large amount of DME directly, from a feed of NH₃ and CO_{X}, wherein x = 1-2. The possibility to perform the DME synthesis under these conditions is further supported by literature evidence of ammonia cracking at these temperatures, and sorption-enhanced DME synthesis.

In addition, it has been demonstrated experimentally that the water removal in the reaction mixture can be reached by a relatively efficient pressure swing operation of the sorption-enhanced reaction system (van Kampen et al., Reaction Chemistry & Engineering, 6(2), 244-257 (2021)). Thus, the current concept allows to directly produce valuable DME, and side product N₂, from a stoichiometric NH₃-CO₂ feed. Finally, although the starting materials for the current simulation were CO₂ and NH₃, the results extend towards CO and NH₃ as starting materials and a combination of CO and CO₂ and NH₃ as starting material. The reverse water-gas shift equilibrium couples CO and CO₂ which provides for a similar outcome regardless of whether CO or CO₂ is present as a starting material (van Kampen, J., Efficient carbon utilization to dimethyl ether by steam adsorption enhancement, PhD Thesis, Technische Universiteit Eindhoven, 2021).

### Example 2 - Conventional NH₃ cracking

Using the modelling approach of example 1 ammonia cracking was simulated:

| | | |
|---|---|---|
| 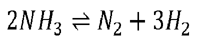 | ammonia cracking | (1) |

So only equation 1 was used and the feed was pure NH₃. Model simulations were performed at two different pressures; 1 bara and 50 bara. The equilibrium mole fraction of the different species is in table 2. At a lower pressure and the same temperature H₂ concentration is higher and NH₃ concentration is lower. At the same pressure and a higher temperature, the H₂ concentration is higher and NH₃ concentration lower. Therefore a low pressure or a high temperature are advantageous for NH₃ cracking.

**Table 2. Product composition for NH₃ cracking at different temperatures and pressures, expressed in mol% on total.**

| | **200°C** | | **300°C** | | **400°C** | |
|---|---|---|---|---|---|---|
| | ***1 bara*** | ***50 bara*** | ***1 bara*** | ***50 bara*** | ***1 bara*** | ***50 bara*** |
| **NH₃** | 13% | 75% | 2% | 42% | 0.4% | 15% |
| **H₂** | 65% | 7% | 72% | 45% | 75% | 63% |

## Claims

1. Process for the conversion of ammonia (NH₃) and CO_{X}, wherein x = 1-2, to dimethyl ether (DME) wherein in a reaction step a) a feed comprising NH₃ and COx, wherein x = 1-2, is provided to a reactor to obtain a reaction product comprising DME, wherein the reactor comprises a catalyst for converting NH₃ to nitrogen (N₂) and hydrogen (H₂), a catalyst for converting H₂ and CO_{X}, wherein x = 1-2, to DME, and a sorbent material capable of adsorbing water.

2. The process according to claim 1 wherein the reaction temperature is in the range of 150°C - 400°C, preferably in the range of 200°C - 300°C.

3. The process according to claim 1 or 2 wherein the reaction pressure is in the range of 1 - 100 bara, preferably in the range of 20 - 80 bara, even more preferably in the range of 30 - 70 bara.

4. The process according to any one of the preceding claims wherein the feed comprises H₂, preferably wherein the feed comprises in the range of 1-20 mol% of H₂ on total of the feed.

5. The process according to any one of the preceding claims wherein the carbon selectivity towards DME is at least 50%, preferably at least 70%, more preferably at least 90%.

6. The process according to any one of the preceding claims, wherein the catalyst for converting NH₃ to N₂ and H₂ is a metal based catalyst, preferably a metal/support catalyst, a metal alloy catalyst, a metal composite catalyst and/or a metal oxide catalyst.

7. The process according to any one of the preceding claims, wherein the catalyst for converting H₂ and CO_{X}, wherein x = 1-2, to DME is selected from a bifunctional catalyst for converting H₂ and CO_{X}, wherein x = 1-2, to DME or a combination of a methanol synthesis catalyst and a methanol dehydration catalyst, preferably the catalyst for converting H₂ and CO_{X}, wherein x = 1-2, to DME is an alumina containing copper/zinc oxide based catalyst.

8. The process according to any one of the preceding claims wherein the reactor comprises in the range of 20 - 90 wt.% of the sorbent material capable of adsorbing water based on total of the catalyst for converting NH₃ to N₂ and H₂, the catalyst for converting H₂ and CO_{X}, wherein x = 1-2, to DME, and the sorbent material capable of adsorbing water.

9. The process according to any one of the preceding claims, wherein the sorbent material capable of adsorbing water is a molecular sieve having a pore diameter in the range of 2-50 Å, preferably in the range of 2-10 Å, even more preferably in the range of 2-5 Å, preferably the sorbent material capable of adsorbing water is a zeolite, more preferably an LTA zeolite.

10. The process according to any one of the preceding claims wherein the process comprises a second step b) after step a) which is a sorbent regeneration step wherein the sorbent material is contacted with a purge gas.

11. The process according to claim 9 or 10 wherein the temperature in step b) is at least 100°C higher than the temperature of step a) and/or wherein the pressure of step b) is at least 10 bar lower than the pressure of step a), preferably wherein the temperature in step b) is at least 150°C higher than the temperature of step a) and/or wherein the pressure of step b) is at least 20 bar lower than the pressure of step a).

12. The process according to any one of claims 9-11, having two or more reactors comprising the catalyst for converting NH₃ to N₂ and H₂, the catalyst for converting H₂ and CO_{X}, wherein x = 1-2, to DME, and the sorbent material capable of adsorbing water, preferably wherein at least one reactor of the two or more reactors is performing step a).

13. Composition comprising a catalyst for converting NH₃ to N₂ and H₂, a catalyst for converting H₂ and CO_{X}, wherein x = 1-2, to DME and a sorbent material capable of adsorbing water.

14. Use of the composition of claim 13 for converting ammonia (NH₃) and COx, wherein x = 1-2, to dimethylether (DME), preferably wherein the use involves one or more of the following:
- the conversion is increased compared to when the sorbent material is not present,
- the reaction rate is increased compared to when the sorbent material is not present, or
- the carbon selectivity towards DME is increased compared to when the sorbent material is not present.

15. A reactor comprising the composition of claim 13, preferably the reactor is a fixed bed reactor or a fluidized bed reactor, more preferably the reactor is a fluidized bed reactor.
